# EUROPEAN PATENT APPLICATION

(11) **EP 1 149 585 A1**
(43) Date of publication of application: **31.10.2001**
(21) Application number: 00401174.8
(22) Date of filing: 27.04.2000
(51) Int. Cl.: A61K 38/46, A61P 1/14

(54) **Use of recombinant gastric lipase for treating functional dyspepsia**

(71) Applicant: WARNER-LAMBERT COMPANY, Morris Plains New Jersey 07950 (US); MERISTEM THERAPEUTICS, 63100 Clermont-Ferrand (FR)
(72) Inventor: Berna, Patrick, 91300 Massy (FR); Milano, Stéphane, 91300 Massy (FR)
(74) Representative: Phélip, Bruno

(57) **Abstract**

The invention relates to the use of recombinant gastric lipase for the preparation of a drug for the treatment of functional dyspepsia.

## Description

The invention relates to the use of recombinant gastric lipase for the preparation of a drug for preventing and/or treating functional dyspepsia. The invention also concerns a method for treating functional dyspepsia comprising administering to a patient in need thereof an effective amount of the above compound.

### Background

Dyspepsia is a very common syndrome that accounts for about 30% of cases seen by gastroenterologists. Functional dyspepsia (also known as non-ulcer dyspepsia or NUD, i.e. without any clear and obvious gastric lesion) is the most important etiologic category and represents about 60% of all dyspepsia cases.

Dyspepsia refers to pain or discomfort centered in the upper abdomen. The symptoms associated with this condition include abdominal fullness, early satiety, bloating, belching, nausea/vomiting and heartburns. Medication has until now been decided based on the main symptom; this however has proven to be unsatisfactory, as there is a substantial overlap between these symptoms. Also, none of the treatments known today, including antacids, H2-receptor antagonists, has proven to be efficient.

Thus, there is a need to find a drug that would be efficient for treating functional dyspepsia. Through satisfactory results for at least some symptoms that would thus lead to a general improvement in the patient's condition.

It should also be taken into consideration that above 75% of dyspeptic individuals do not seek medical attention, rendering this need acute.

### Summary of the invention

The invention concerns the use of recombinant gastric lipase for the preparation of a medicament useful in the treatment of functional dyspepsia.

More particularly, the use of the recombinant gastric lipase is indicated in pathologies such as those comprising dysmotility-like dyspepsia, ulcer-like dyspepsia, reflux-like dyspepsia and non-specific dyspepsia.

In addition, the invention concerns the use of recombinant gastric lipase for improving prandial and/or postprandial fundus accommodation as well as gastric emptying of solids and/or liquids.

The recombinant gastric lipase involved in the present invention is preferably a recombinant dog gastric lipase.

For its use according to the invention the medicament comprises from 7mg to about 660mg, preferably from about 30mg to about 330mg of recombinant gastric lipase.

The invention is disclosed in more details in the following specification.

### Detailed description of the invention

Recombinant gastric lipase has been described in WO-A-9413816, WO-A-9633277, WO-A-9310243 and EP0191061, the contents of these documents being incorporated herein by reference. Preferably, the recombinant gastric lipase is recombinant dog (or canine) gastric lipase such as disclosed in WO-A-9413816 (such as published with the request for correction and incorporating it) . The recombinant gastric lipase disclosed in WO-A-9413816 is notably intended to facilitate the absorption of fat ingested by an individual suffering from one or more pathologies affecting the level of production of lipases.

The above lipase is usually provided as a pharmaceutical composition comprising the lipase, optionally with at least one pharmaceutically acceptable carrier or excipient or stabilizer. The lipase can be available e.g. as a powder, which can be obtained by freeze-drying. For preparing pharmaceutical compositions from the lipase used in this invention, inert, pharmaceutically acceptable carriers can be either solid or liquid.

Solid form preparations include powders, tablets, dispersible granules, capsules and cachets.

A solid carrier can be one or more substances which may also act as diluents, flavoring agents, solubilizers, lubricants, suspending agents, binders, or tablet disintegrating agents; it can also be an encapsulating material. In powders, the carrier is a finely divided solid, which is in a mixture with the finely divided lipase. In tablets, lipase is mixed with the carrier having the necessary binding properties in suitable proportions and compacted in the shape and size desired. The powders, tablets, cachets or encapsulated forms for capsules preferably contain from about 5% to about 70% of lipase. Suitable carriers are magnesium carbonate, magnesium stearate, talc, lactose, sugar, pectin, dextrin, starch, tragacanth, methyl cellulose, sodium carboxymethyl cellulose, a low-melting wax, and the like.

As stabilizer, it should be understood any substance which stabilizes the biological activity of the lipase and particularly during the freeze-drying step and the subsequent storage, such as mannitol, lactose, Nacl, trealose or saccharose.

Tablets, powders, cachets, and capsules can be used as solid dosage forms suitable for oral administration.

Liquid form preparations include solutions, suspensions, and emulsions.

Aqueous solutions for oral administration can be prepared by dissolving the lipase in water and adding suitable flavoring agents, stabilizers, buffers and thickening agents as desired. Aqueous suspensions for oral use can be made by dispersing the finely divided lipase in water together with a viscous material such as natural synthetic gums, resins, methyl cellulose, sodium carboxymethyl cellulose, and other suspending agents known to the pharmaceutical formulation art.

Preferably the pharmaceutical preparation is in unit dosage form. In such form, the preparation is divided into unit doses containing appropriate quantities of lipase. The unit dosage form can be a packaged preparation, the package containing discrete quantities of the preparation, for example, packaged tablets, capsules, and powders in vials or ampoules. The unit dosage form can also be a capsule, cachet, or tablet itself, or it can be the appropriate number of any of these packaged forms.

The dosage form will be such that the amount of recombinant gastric lipase administered will generally be from about 20 mg to about 2000 mg per day. Preferred doses will be from about 100 mg to about 1000 mg per day (for an adult with average weight of 70 kg). Preferably, the composition will be taken at the time of the meal; the dosage will thus be about 7 mg to about 660 mg, preferably from about 30 mg to about 330 mg.

When the recombinant gastric lipase is used for treating functional dyspepsia according to the invention functional dyspepsia covers notably ulcer-like dyspepsia, dysmotility-like dyspepsia, reflux-like dyspepsia and non-specific dyspepsia.

Ulcer-like dyspepsia refers notably to dyspepsia where the main symptom is epigastric pain.

Dysmotility-like dyspepsia refers notably to dyspepsia where the main symptom is an unpleasant or troublesome non-painful sensation (discomfort) centered in the upper abdomen; this sensation may be characterized by or associated with upper abdominal fullness, early satiety, bloating or nausea.

Reflux-like dyspepsia refers notably to dyspepsia where the main symptom is heartburn, but where the reflux is without esophagitis. It shall be understood that GERD (gastro-esophageal reflux disease) is excluded from functional dyspepsia.

Non-specific dyspepsia refers notably to functional dyspepsia that does not fall in any of the above categories.

The invention is particularly useful for the treatment of reflux-like dyspepsia, non-specific dyspepsia and especially dysmotility-like dyspepsia.

The invention can be applied to any mammal, including human.

The invention also provides a method for the treatment of functional dyspepsia comprising administering to a mammal, preferably a human, in need thereof an effective amount of recombinant gastric lipase.

In the case of dysmotility-like dyspepsia, symptoms associated with impaired postprandial fundus accommodation and/or gastric empty are often seen in patients suffering from said dyspepsia.

Accordingly some symptoms seen in dyspepsia may be due to an ileal brake which results for the patients in a lack of prandial and postprandial fundus accommodation. In other words, there is a lack of compliance of the stomach volume to the meal volume. The gastric recombinant lipase allows improving deficient fundus accommodation.

The gastric emptying, associated or not with other symptoms, can appear with solids and/or liquids, as this has been already reported in the literature.

The major advantage of the use of recombinant gastric lipase for the invention is its acido-resistance to low pH and particularly to gastric pH. Hence, this lipase is immediately efficient as soon as it has been ingested thereby allowing improvement of symptoms associated with impaired postprandial fundus accommodation and/or gastric emptying particularly and of dyspepsia in general.

The following examples illustrate the invention without limiting it.

### Example 1. Effect of recombinant gastric lipase on the Fundus accommodation

The protocol of this study is similar to the protocol disclosed in Azpiroz et al., Am. J. Physiol. (1985), 248, G229-G237 as well as in Am. J. Physiol. (1985), 249, G501-G509. 6 Mongrel female dogs are used in the present study. This protocol makes use of a specific gastric barostat. The electronic barostat is an instrument that maintains a determined constant pressure within an air-filled intragastric bag. When the stomach contracts, the barostat aspires air to maintain a constant pressure within the bag; when the stomach relaxes, air is injected. The system measures gastric tone by recording changes in the intragastric volume of air. In brief, the barostat consists of a strain gauge linked by an electronic relay to an air-injection system. This system is activated to inject air when the pressure at the strain gauge is less than a preset pressure (in this example about + 2 mm Hg) and to withdraw air when pressure is more than the preset pressure. The bag, made of ultrathin polyurethane, has a 700 ml capacity and is connected to the strain gauge and to the injection system by a tube such as a double-lumen French no.14 polyvinyl tube.

The meal is administered according to the following route:

The dogs are given about 200 ml of solid and/or liquid normal or fat enriched food (see table 1) that is ingested by the normal route. The dogs are trained to eat with the barostat system in position. The food with recombinant gastric lipase is obtained by merely adding recombinant gastric lipase in an amount of about 30 mg to 330 mg to the above meal.

Barostat and manometry data are recorded in accordance with the protocol as disclosed in the Azpiroz publication.

### Example 2. Effects of the recombinant gastric lipase on Gastric emptying.

The protocol of this study is comparable to the protocol disclosed in Tanaka et al., Am. J. Physiol. (1997), 272, G909-G915. 6 Mongrel female dogs are used in the present study. Two test meals are given, after measurement of their freeze-dried weight, and 1,5 ml duodenal samples are collected through an indwelling tube at 15 min intervals, and their absolute weight is measured. The duodenum is continuously perfused with phenolsulfonphthalein to determine the recovery of each sample. The test meal comprises fat, carbohydrate, proteins according to the weight ratio as disclosed in table 1. The meal with recombinant gastric lipase is obtained by merely adding recombinant gastric lipase in an amount of about 30 mg to 330 mg to the above meal. The gastric emptying of the solids and/or the liquids is determined as the half-emptying times (expressed in hr)

**TABLE 1**

| Enriched test meal (190 ml) | | | | |
|---|---|---|---|---|
| | TEST MEAL | % | WEIGHT(g) | CALORIES |
| | | | | |
| | 190 | | | |
| Water | | 9 | 17.1 | |
| Fats | | 7 | 13.3 | 119.7 |
| Proteins | | 23 | 43.7 | 131.1 |
| Fibers | | 4.8 | 9.12 | ND |
| | | | | |
| TOTAL | | | | 250.8 Kcal |
| + olive oil | | | 10 | 90 Kcal |
| | | | | 340.8 Kcal |
| | | | | |

| Normal test meal (250 ml) | | | | |
|---|---|---|---|---|
| | | | | |
| | TEST MEAL | % | WEIGHT(g) | CALORIES |
| | | | | |
| | 250 | | | |
| Water | | 9 | 22.5 | |
| Fats | | 7 | 17.5 | 157.5 |
| Proteins | | 23 | 57.5 | 172.5 |
| Fibers | | 4.8 | 12 | ND |
| | | | | |
| TOTAL | | | | 330 Kcal |
| | | | | |
| Ration permeability: 50 ml NaCl 0.9% comprising 7.5 g PEG 3350 | | | | |

## Claims

1. Use of recombinant gastric lipase for the preparation of a medicament for the treatment of functional dyspepsia.

2. Use according to claim 1, in which the functional dyspepsia comprises dysmotility-like dyspepsia.

3. Use according to claim 1, in which the functional dyspepsia comprises ulcer-like dyspepsia.

4. Use according to claim 1, in which the functional dyspepsia comprises reflux-like dyspepsia.

5. Use according to claim 1, in which the functional dyspepsia comprises non-specific dyspepsia.

6. Use according to claim 1 or 2, for improving prandial and/or postprandial fundus accommodation.

7. Use according to claim 1 or 2, for improving gastric emptying of solids and/or liquids.

8. Use according to any one of claims 1 to 7, in which the recombinant gastric lipase is recombinant dog gastric lipase.

9. Use according to any one of claims 1 to 8, in which the drug comprises from 7 mg to about 660 mg, preferably from about 30 mg to about 330 mg of recombinant gastric lipase.
